Europäisches Patentamt

European Patent Office

Office européen des brevets

(11) Publication number: **0 256 653**
**A2**

## EUROPEAN PATENT APPLICATION

(21) Application number: **87306004.0**

(22) Date of filing: **07.07.87**

(51) Int. Cl.⁴: **A61M 1/36**

(30) Priority: **10.07.86 US 883976**

(43) Date of publication of application:
**24.02.88 Bulletin 88/08**

(84) Designated Contracting States:
**AT BE CH DE FR GB IT LI NL SE**

(71) Applicant: **HAEMONETICS CORPORATION**
**400 Wood Road**
**Braintree Massachusetts 02184(US)**

(72) Inventor: **Verkaart, Wesley H.**
**25 Cross Street**
**Duxbury Massachusetts 02332(US)**

(74) Representative: **Harvey, David Gareth et al**
**Graham Watt & Co. Riverhead**
**Sevenoaks Kent TN13 2BN(GB)**

(54) **Blood compatible heat exchanger.**

(57) A blood compatible, sterilizable, heat exchanger
(10) comprises a finned inner tube (30) and an outer
tube (20) which are spaced apart to provide an
annular fluid-flow space (60); the inner tube provides
a second fluid flow space. End caps (40I, 40E) are
fitted to the assembled tubes (20, 30) and are sealed
(a) to the outer tube, and (b) to the inner tube via
gaskets (50); the end caps so fit the tubes (20, 30)
as to prevent relative movement between said tubes.
The caps have respective inlets or outlets (42) for
fluid to enter and leave the annular flow space (60).
In use, heat exchange fluid may be passed through
the inner tube, when blood is passed in counter-flow
direction through the space (60).

Fig. 1

EP 0 256 653 A2

## "BLOOD COMPATIBLE HEAT EXCHANGER"

This invention relates to a blood compatible heat exchanger.

When blood cells are transfused into the body, it is highly desirable that they be at a temperature that will not cause a shock on the system or themselves as they attain natural body temperature. Conventional heat exchange technologies for altering blood temperature have proven less than ideal in meeting the requirements of performance, compatibility, and sterility demanded by current surgical procedures. Conventional tube-within-a-tube heat exchangers lack the ability to securely lock the two tubes together to prevent unsterilized sections from contacting a sterile zone.

Delahaye et al. (U.S. Patent No. 3,998,477) describes a method of forming a non-rigid connection between two circular pipes utilizing a bulb-shaped zone on the inner pipe used in conjunction O-Rings and double-lipped gaskets. However, being non-rigid, his design may not be suitable for maintaining a sterile environment between the tubes. Furthermore, Delahaye at no time specifies that his design may be used for heating blood, recommending instead that it be used in the construction of photochemical reactors.

Current blood-compatible heat exchangers use a series of plates or fins, often constructed of stainless steel, within a tube through which the various fluids pass. By contacting these fins, the fluid is either heated or cooled depending on the temperature of the fin. The ends of the device are sealed with a rubbery sealant. Disadvantages of this type of exchanger include a lower than desirable efficiency as well as a large priming volume. Smaller priming volumes are preferred, because when operating with a fluid such as blood, it is desirable to minimize the amount of fluid needed to fill the device so that heat exchange may commence as quickly and efficiently as possible.

### Summary of the Invention

This invention relates to a sealable tube-within-a-tube heat exchanger that has high efficiency, low priming volume, can withstand a continuous pressure of 10 psi (0.69 bar) between the tubes, is blood compatible, is ETO sterilizable with a minimum residual and short required aeration time, can maintain sterility indefinitely in a closed system, and is easy to assemble correctly and difficult to assemble incorrectly.

More particularly, this invention relates to a blood compatible heat exchange apparatus for cooling or heating blood comprising an outer, elongate, longitudinally cylindrical, optically clear polycarbonate tube within which is disposed an inner, elongate, longitudinally cylindrical tube thereby providing a transverse, thermally conductive path or heat exchange region for coolant fluid or heating fluid passing longitudinally between the tubes. The inner tube is provided with a plurality of helical, radially outward extensions along the longitude thereof. An inlet cap is provided at one longitudinal end of the tubes and an outlet cap is provided at the opposite longitudinal end. Each end cap has a shoulder which abuts the outer tube, an end bore through which the inner tube passes and a transverse aperture for providing fluid communication for introducing blood from the outside of the apparatus into the space between the inner surface of the outer tube and the outer, helical surface of the inner tube to facilitate thermal exchange between the fluid in the inner tube and the blood in the space between the inner and outer tubes.

This invention also relates to a heat exchange apparatus comprising an outer, elongate cylindrical tube within which is disposed an inner, elongate cylindrical tube having a pair of circumferential grooves about the periphery, one adjacent each end, each groove containing a flexible torroidal gasket. An inlet end cap is located at one longitudinal end of the tubes and an outlet end cap is located at the opposite end. Each end cap contains a longitudinal transverse bore with an inner diameter which permits the end cap to slide over each gasket and compress it. Furthermore, each end cap is provided with an outer shoulder which abuts the gaskets and an inner shoulder which abuts the outer tube and prevents it from entering the entire length of the transverse bore in the end cap. The end caps serve to retain the inner and outer tubes in a stationary, spaced apart relationship, thus forming a heat transfer region between the outer surface of the inner tube and the inner surface of the outer tube. Finally each end cap also contains a transverse aperture allowing fluid to enter the inner cap, flow through the heat exchange region and exit the transverse aperture in the outlet end cap.

The invention will now be described in more detail by way of example only with reference to the accompanying drawings, in which:

Figure 1 is a schematic diagram of apparatus according to the invention and

Figure 2 is a cutaway side view of an end cap used in the apparatus.

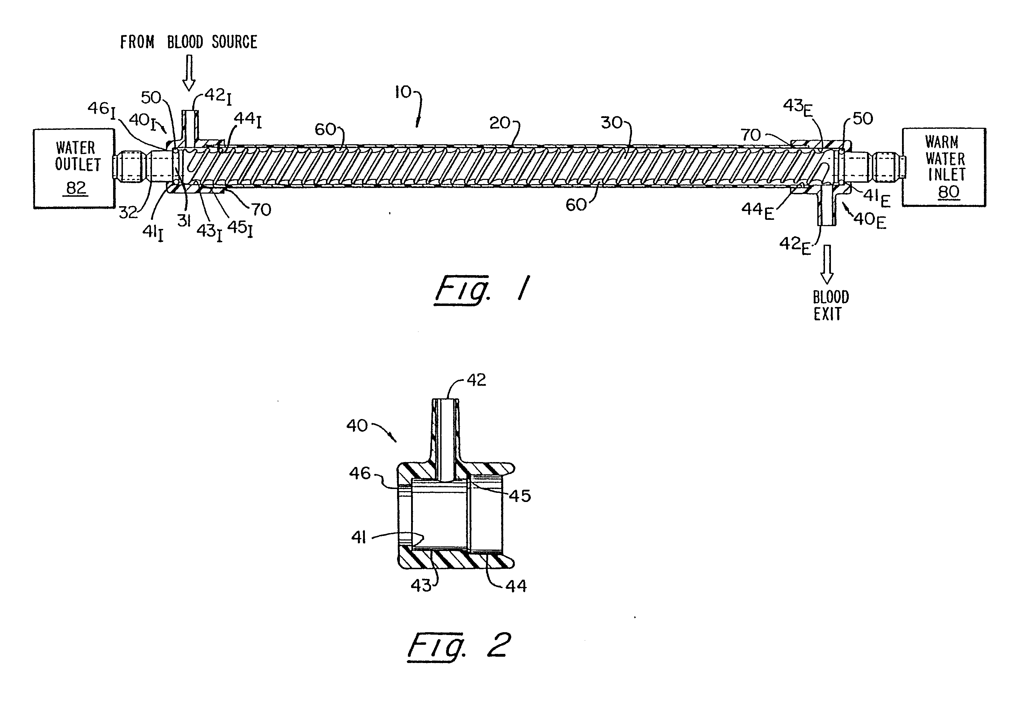

Figure 1 is a schematic drawing of a sealable tube-within-a-tube heat exchange apparatus 10. An outer tube 20, surrounds an inner tube 30, with the apparatus being held together in a spaced apart relationship by inlet and outlet end caps 40I and 40E and sealed by flexible torroidal gaskets such as O-Rings 50. The outer tube 10, may be any tough, blood-compatible material capable of withstanding an internal pressure of 10 psi (0.69 bar) for extended time periods. Clear polycarbonate tubing is preferred for this application. The inner tube 30, preferably aluminum that has a natural hardcoat finish, is disposed in a coaxial relationship with the outer tube 20, and extends longitudinally from each end of the outer tube 20.

In the preferred embodiment, the inner tube 30, is formed of a plurality of spiral, fin-like extensions along the length of the tube. These extensions serve to provide a greater surface area per unit length on the inner tube 30, and thus, more extensive contact between the blood and the inner tube 30. As such, these fins provide better heat transfer per unit length between the blood and the liquid contained within the inner tube.

Securing the tube assembly are end caps 40I and 40E, preferably made of polycarbonate. Flexible torroidal gaskets 50, preferably O-Rings made of medical grade silicon rubber seated in grooves seal the apparatus and prevent relative movement between the parts. Each end cap has a first shoulder 41I or 41E, which opposes a circumferential groove 31, at the periphery of each end of the inner tube 30. The O-Rings 50, are seated in the circumferential grooves 31. The O-Rings being thus interposed between opposing shoulder 41I ro 41E, and groove 31, seal the assembly and lock it in place. By preventing relative longitudinal movement between the coaxially disposed tubes 20 and 30, unsterilized inlet and outlet regions 32 of the inner tube 30 are prevented from contacting the sterile pathway 60, between the tubes 20 and 30, and contaminating it. A solvent bond or weld 70, is used to secure each end cap, to the outer tube 20.

Figure 2 is a cutaway side view of an end cap 40, in much greater detail. The end cap is provided with an aperture 42, which allows communication between a blood inlet or outlet tube and the interior of the apparatus. Aperture 42, extends into a first longitudinal transverse opening or bore 43, which has at one end a first circular shoulder 41, against which an O-Ring will seat and at the other end, a second circular shoulder 45, which forms the end of a second longitudinal transverse opening or bore 44, abutting and communicating with the first. The first shoulder 41, defines an aperture 46, through which the inner tube extends.

The end cap 40, is attached to the outer tube by the means of a solvent bond or weld, 70 as indicated in Figure 1. The outer tube enters the second longitudinal, transverse opening 44, and abuts the second shoulder 45. This shoulder prevents the outer tube from entering the first longitudinal transverse opening 43, blocking the aperture 42, which would prevent blood flow into or out of the device.

The apparatus of Figure 1 operates as follows. A warm water supply 80 is attached to one end of the inner tube 30, which extends through end cap 40E, at the water inlet region 32. A water outlet 82 is attached to the extending section 32 of the inner tube 30 at the opposite end of the apparatus 10. A blood input source is attached to the aperture 42I, adjacent the water outlet side of the apparatus 10. The blood enters the longitudinal, transverse opening 43I, flows through the longitudinal, transverse opening 44I, and enters the sterile pathway 60, while constantly contacting the warm-water containing inner tube 30, thus establishing countercurrent heat exchange. The blood enters the logitudinal transverse opening 44E, at the opposite end cap 40E, flows into the second longitudinal transverse opening 43E, and exits through the aperture 42E, at the water inlet side of the device.

As the apparatus contains a minimal number of parts it is very easy to assemble, thus making incorrect assembly very unlikely. Furthermore, if the individual parts are comprised of the materials described as preferred, the devide is blood compatible as well as being ETO sterilizable with minimum residual and short aeration time.

The embodiment described herein is the presently preferred embodiment of a blood compatible heat exchanger with an axial lock, O-Ring seal. It is understood however that various modifications may be made to the embodiment described herein by those skilled in the art without departing from the scope of the invention as is defined by the claims set forth hereinafter.

## Claims

1. A blood compatible heat exchange apparatus for cooling or heating blood which comprises:

(a) an outer, elongate, longitudinally cylindrical, optically clear polycarbonate tube (20);

(b) an inner, elongate, longitudinally cylindrical tube (30) disposed within said outer tube for providing a transverse thermally conductive path for coolant or heating fluid passing longitudinally through said tube (30), the inner tube (30) having a plurality of helical, radially outward extensions along the longitude thereof; and

(c) an inlet cap (40I) at one longitudinal end of said tubes (20, 30) and an outlet cap (40E) at the opposite ends, each end cap having a shoulder (45) which abuts the outer tube (20), an end bore (46) through which the inner tube (30) passes and a transverse aperture (42) for providing fluid communication for introducing blood from the outside of the apparatus into the space (60) between the inner surface of the outer tube (20) and the outer helical surface of the inner tube (30) to facilitate thermal exchange between fluid in the inner tube and blood in the space (60) between the inner and outer tubes.

2. The heat exchanger according to claim 1, wherein said end caps (40I,E) retain the inner and outer tubes (20, 30) in a fixed, spaced apart relationship, thereby forming a heat transfer region (60) between the outer surface of the inner tube and the inner surface of the outer tube.

3. The heat exchanger according to claim 1 or claim 2, wherein the transverse aperture (42I) of said end caps allows blood to enter the inlet cap, and to exit through the transverse aperture in the outlet cap after flow through the spaced apart heat exchange region.

4. The heat exchanger according to claim 1, 2 or 3, wherein the outer tube (20) extends along the entire fluid pathway from the inlet cap (40I) to the outlet cap (40E).

5. The heat exchanger according to any of claims 1 to 4, wherein the helical, radially outward extensions on the inner tube (30) are in contact with the inner surface of the outer tube (20), thereby creating a series of helical flow paths in the heat transfer region (60) of the apparatus.

6. The heat exchanger according to any of claims 1 to 5, which is capable of (a) maintaining a continuous pressure between the inner tube and the outer tube of 10 psi (0.69 bar), and/or (b) being ETO sterilised and/or (c) maintaining sterility in a closed system for an indefinte time period.

7. The heat exchanger according to any of claims 1 to 6, wherein said inner tube (30) is constructed of thermally conductive metal such as aluminium, for example aluminium with a natural hardcoat finish.

8. A heat exchange apparatus which comprises:

(a) an outer, elongate, cylindrical tube (20);

(b) an inner elongate, cylindrical tube (30), optionally made of metal, disposed within said outer tube and having a pair of circumferential grooves (30) about the periphery, one adjacent each end;

(c) a pair of flexible torroidal gaskets (50), one each being disposed in said groove (31) at each end of said inner cylindrical tube; and,

(d) an inlet cap (40I) at one longitudinal end of said tubes and an outlet cap (40E) at the opposite end, each having a first shoulder (41) which abuts the corresponding gasket (50) and a second shoulder (45) which abuts the outer tube, either or both of the caps (40I, E) being for example made of plastic.

9. The apparatus according to claim 8, wherein the end caps (40I, E) retain the inner and outer tubes (20, 30) in a fixed, spaced apart relationship forming a heat transfer region (60) between the outer surface of the inner tube and the inner surface of the outer tube.

10. The apparatus according to claim 8 or claim 9, wherein the end caps each have a transverse bore (43) with an inner diameter which enables the end cap to slide over a gasket (50) and compress it.

11. The apparatus according to claim 8, 9 or 10, wherein the end caps each have a transverse aperture (42) allowing a fluid to enter the inlet cap and to exit the outlet cap after flowing through a heat exchange region (60) between the tubes (20, 30).

12. A heat exchanger according to any of claims 8 to 11, wherein each end cap is sealed to a respective end of said outer tube (20).

13. A heat exchange apparatus which comprises:

(a) an outer tube (20);

(b) an inner tube (30) disposed within said outer tube and having a pair of circumferential grooves (31) about the periphery, one adjacent each end;

(c) a pair of gaskets (50) disposed one in each of the said grooves (31) of the inner tube; and

(d) an inlet cap (40I) at one longitudinal end of said tubes and an outlet cap (40E) at the opposite end, each having a bore (43) with an inner diameter which permits the respective cap to slide over and compress the associated gasket, an outer shoulder (41) on each said cap which abuts the gasket (50) and an inner shoulder (45) which abuts the outer tube (20), said end caps serving to retain the tubes in a fixed, spaced apart relationship, and each of said end caps further having an aperture allowing a fluid to enter the inlet end cap, flow through the spaced apart region and exit the aperture in the outlet end cap.

14. A heat exchange apparatus according to any of claims 8 to 13, which comprises an outer, elongate, cylindrical tube consisting essentially of a blood compatible, polycarbonate resin; an inner, elongate, cylindrical tube consisting essentially of blood compatible aluminium in the circumferential grooves of whcih are seated the said gaskets (50) which consist essentially of medical grade, silicone rubber, and the said caps (40I, 40E) each consist essentially of a blood compatible, polycarbonate resin and have a longitudinal, transverse bore (43) sized to permit the end cap to slide over an associated gasket and compress it, an outer shoulder (41) on each said end cap to abut the gasket and an inner shoulder (45) to abut the outer tube and prevent it from entering the entire length of the transverse bore in the end cap, said end caps serving to retain the inner and outer tubes (20, 30) in a fixed, spaced apart relationship forming a heat transfer region between the outer surface of the inner tube and the inner surface of the outer tube, each of said end caps further having a transverse aperture (42) allowing a fluid to enter the inlet cap, flow through the heat exchange region (60) between the spaced apart tubes and exit the transverse aperture in the outlet cap, and the said end caps being tightly and rigidly sealed to the outer peripheral surface of the outer tube adjacent each end thereof.

FROM BLOOD SOURCE

*Fig. 1*

*Fig. 2*

0 256 653